# EUROPEAN PATENT SPECIFICATION

(11) **EP 4 561 481 B1**
(45) Date of publication and mention of the grant of the patent: **26.08.2026**
(21) Application number: 23757471.0
(22) Date of filing: 25.07.2023
(51) Int. Cl.: A61B 18/26, G02B 6/38

(54) **ROUTING ASSEMBLY FOR INTRAVASCULAR LITHOTRIPSY CATHETER SYSTEM**
FÜHRUNGSANORDNUNG FÜR INTRAVASKULÄRES LITHOTRIPSIE-KATHETERSYSTEM
ENSEMBLE DE ROUTAGE POUR SYSTÈME DE CATHÉTER DE LITHOTRIPSIE INTRAVASCULAIRE

(30) Priority: 26.07.2022 US 202263392342 P; 07.08.2022 US 202263395853 P; 24.07.2023 US 202318357947
(43) Date of publication of application: 04.06.2025
(60) Divisional application: 26193880.7
(73) Proprietor: Boston Scientific Scimed, Inc., Maple Grove, Minnesota 55311 (US)
(72) Inventor: SCHULTHEIS, Eric, San Clemente, CA 92673 (US); SALINAS, Alvin, San Marcos, CA 92069 (US); DAHL, Peter, Riverside, CA 92506 (US)
(74) Representative: Vossius & Partner Patentanwälte Rechtsanwälte mbB
(86) International application number: PCT/US2023/070970
(87) International publication number: WO 2024/026322

(56) References cited:
- US-A- 5 764 843
- US-A1- 2019 072 378
- US-A1- 2021 137 598

## Description

### BACKGROUND

Vascular lesions within vessels in the body can be associated with an increased risk for major adverse events, such as myocardial infarction, embolism, deep vein thrombosis, stroke, and the like. Severe vascular lesions, such as severely calcified vascular lesions, can be challenging to treat and achieve patency for a physician in a clinical setting.

Vascular lesions may be treated using interventions such as drug therapy, balloon angioplasty, atherectomy, stent placement, and vascular graft bypass, to name a few. Such interventions may not always be ideal or may require subsequent treatment to address the lesion.

Intravascular lithotripsy is one method that has been recently used with some success for breaking up vascular lesions within vessels in the body. Intravascular lithotripsy utilizes a combination of pressure waves and bubble dynamics that are generated intravascularly in a fluid-filled balloon catheter. In particular, during an intravascular lithotripsy treatment, a high energy source is used to generate plasma and, ultimately, pressure waves as well as a rapid bubble expansion within a fluid-filled balloon to crack calcification at a treatment site within the vasculature that includes one or more vascular lesions. The associated rapid bubble formation from the plasma initiation and resulting localized fluid velocity within the balloon transfers mechanical energy through the incompressible fluid to impart a fracture force on the intravascular calcium, which is opposed to the balloon wall. The rapid change in fluid momentum upon hitting the balloon wall is known as hydraulic shock, or water hammer.

There is an ongoing desire to enhance vessel patency and optimization of therapy delivery parameters within an intravascular lithotripsy catheter system in a manner that is relatively easy to control and is consistently manufacturable.

US 2019/072378 A1 discloses, according to its abstract, a "shape sensor system", including "a braided structure in which small diameter bending members including two or more optical fibers, provided with detection target portions, are spirally wound around a core member as an axis in directions opposite to each other, or a braided structure in which three or more small diameter bending members including an optical fiber and a dummy optical fiber or a thin metal wire are braided, a plurality of detection target portions are distributed in the direction around the axis of the core member, the bending directions of the respective bending members are synthesized to detect a bending shape of the probe portion, a function of adjusting the fiber length in a braiding cycle is performed, and position deviation does not occur".

### SUMMARY

The present invention is defined by the appended claims.

The present disclosure is directed toward a catheter system for treating a treatment site within or adjacent to a vessel wall or a heart valve within a body of a patient. In various examples, the catheter system includes a first energy guide, a second energy guide, and a routing assembly. The routing assembly defines a routing space that retains a routing length of each of the energy guides. The routing assembly includes an inner routing guide that is positioned within the routing space. The routing length of each of the energy guides is positioned at least partially about the inner routing guide. The routing space is configured so that the routing length of at least one of the energy guides is adjustable.

In certain examples, each of the energy guides includes an optical fiber.

Each of the energy guides includes a guide distal end and a guide proximal end.

In some examples, the guide proximal end is fixed relative to the routing assembly.

The guide distal end is movable relative to the routing assembly.

In various examples, each energy guide is positioned at least approximately 90 degrees about the inner routing guide.

In some examples, the catheter system further includes an adapter assembly that houses the inner routing guide.

In certain examples, the catheter system further includes an electrical cable that is positioned about the inner routing guide.

In various examples, each energy guide includes an optical guide that protects each energy guide.

In some examples, the catheter system further includes a handle assembly that is usable by a user to selectively position the energy guides near the treatment site, the routing assembly being positioned outside of the handle assembly.

In certain examples, the catheter system further includes a connector assembly that brings each of the energy guides closer together so that the energy guides are in a more compact form, the routing assembly being positioned within the connector assembly.

The present disclosure is also directed toward a catheter system for treating a treatment site within or adjacent to a vessel wall or a heart valve within a body of a patient. In various examples, the catheter system includes a first energy guide and a second energy guide, a handle assembly, and a routing assembly. The handle assembly is usable by a user to selectively position the energy guides near the treatment site. The routing assembly defines a routing space that retains a routing length of each of the energy guides. The routing assembly is positioned outside of the handle assembly. The routing assembly includes an inner routing guide that is positioned within the routing space. The routing length of each of the energy guides is positioned at least partially about the inner routing guide. The routing space is configured so that the routing length of at least one of the energy guides is adjustable.

In some examples, the catheter system further comprises a connector assembly that brings each of the energy guides closer together so that the energy guides are in a more compact form, the routing assembly being positioned within the connector assembly.

In various examples, each of the energy guides includes an optical fiber.

In certain examples, each of the energy guides includes a guide distal end and a guide proximal end.

In some examples, the guide proximal end is fixed relative to the routing assembly.

In various examples, the guide distal end is movable relative to the routing assembly.

In certain examples, each of the energy guides is positioned at least approximately 90 degrees about the inner routing guide.

In some examples, the catheter system further comprises an adapter assembly that houses the inner routing guide.

The present disclosure is also directed toward a catheter system for treating a treatment site within or adjacent to a vessel wall or a heart valve within a body of a patient. In various examples, the catheter system includes a first energy guide and a second energy guide, a handle assembly, a connector assembly, and a routing assembly. The handle assembly is usable by a user to selectively position the energy guides near the treatment site. The connector assembly brings each of the energy guides closer together so that the energy guides are in a more compact form. The routing assembly defines a routing space that retains a routing length of each of the energy guides. the routing assembly is positioned outside of the handle assembly. The routing assembly includes an inner routing guide that is positioned within the routing space. The routing length of each of the energy guides is positioned at least partially about the inner routing guide. The routing space is configured so that the routing length of at least one of the energy guides is adjustable. Each of the energy guides is positioned at least approximately 90 degrees about the inner routing guide.

This summary is an overview of some of the teachings of the present Application and is not intended to be an exclusive or exhaustive treatment of the present subject matter. Further details are found in the detailed description and appended claims. Other aspects will be apparent to persons skilled in the art upon reading and understanding the following detailed description and viewing the drawings that form a part thereof, each of which is not to be taken in a limiting sense. The scope herein is defined by the appended claims.

### BRIEF DESCRIPTION OF THE DRAWINGS

The novel features of this invention, as well as the invention itself, both as to its structure and its operation, will be best understood from the accompanying drawings, taken in conjunction with the accompanying description, in which similar reference characters refer to similar parts, and in which:
Figure 1 is a simplified schematic cross-sectional view of an embodiment of a catheter system in accordance with various embodiments, the catheter system including a handle assembly having features of the present invention;
Figure 2A is a simplified schematic view of a portion of an embodiment of the catheter system, including a handle assembly and an adapter assembly;
Figure 2B is a simplified schematic cross-sectional view of the embodiment of the adapter assembly in Figure 2A, including a routing assembly;
Figure 3 is a perspective view of an embodiment of an optical guide for use in the catheter system; and
Figure 4A is a simplified schematic cross-sectional view of an embodiment of a connector assembly, including the routing assembly;
Figure 4B is a simplified schematic cross-sectional view of the routing assembly in Figure 4A;
Figure 4C is a cross-sectional view of the routing assembly taken on lines 4C-4C in Figure 4B; and
Figure 5 is a simplified top view illustration of an embodiment of the connector assembly.

While embodiments of the present invention are susceptible to various modifications and alternative forms, specifics thereof have been shown by way of examples and drawings, and are described in detail herein. It is understood, however, that the scope herein is not limited to the particular embodiments described.

### DESCRIPTION

Treatment of vascular lesions can reduce major adverse events or death in affected subjects. As referred to herein, a major adverse event is one that can occur anywhere within the body due to the presence of a vascular lesion. Major adverse events can include, but are not limited to, major adverse cardiac events, major adverse events in the peripheral or central vasculature, major adverse events in the brain, major adverse events in the musculature, or major adverse events in any of the internal organs.

As used herein, the terms "treatment site, "intravascular lesion," and "vascular lesion" are used interchangeably unless otherwise noted. As such, the intravascular lesions and/or the vascular lesions are sometimes referred to herein simply as "lesions."

Those of ordinary skill in the art will realize that the following detailed description of the present invention is illustrative only and is not intended to be in any way limiting. Other embodiments of the present invention will readily suggest themselves to such skilled persons having the benefit of this disclosure. Reference will now be made in detail to implementations of the present invention, as illustrated in the accompanying drawings. The same or similar nomenclature and/or reference indicators will be used throughout the drawings, and the following detailed description to refer to the same or like parts.

In the interest of clarity, not all of the routine features of the implementations described herein are shown and described. It is appreciated that in the development of any such actual implementation, numerous implementation-specific decisions must be made in order to achieve the developer's specific goals, such as compliance with application-related and business-related constraints, and that these specific goals will vary from one implementation to another and from one developer to another. Moreover, it is recognized that such a development effort might be complex and time-consuming, but would nevertheless be a routine undertaking of engineering for those of ordinary skill in the art having the benefit of this disclosure.

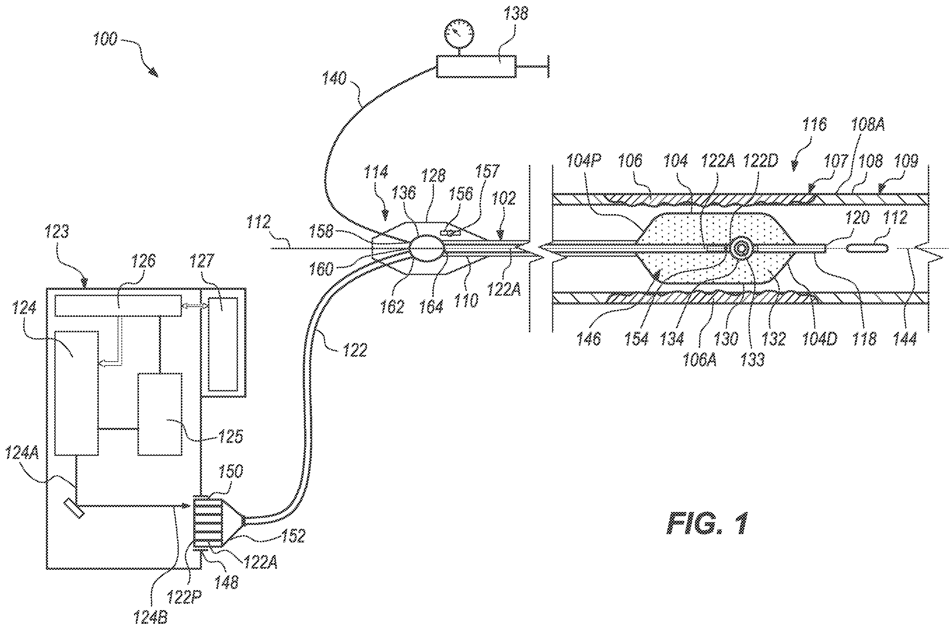

The catheter systems disclosed herein can include many different forms. Referring now to Figure 1, a simplified schematic cross-sectional view illustration is shown of a catheter system 100 in accordance with various embodiments. The catheter system 100 is suitable for imparting pressure waves to induce fractures in one or more vascular lesions within or adjacent to a vessel wall of a blood vessel or on or adjacent to a heart valve within a body of a patient. In the embodiment illustrated in Figure 1, the catheter system 100 can include one or more of a catheter 102, an energy guide bundle 122 including one or more energy guides 122A, a fluid pump 138, a system console 123 including one or more of an energy source 124, a power source 125, a system controller 126, and a graphic user interface 127 (a "GUI"), and a handle assembly 128 that includes a source manifold 136 integrated therein. Alternatively, the catheter system 100 can include more components or fewer components than those specifically illustrated and described in relation to Figure 1.

The catheter 102 is configured to move to the treatment site 106 within or adjacent to a vessel wall 108A of a blood vessel 108 within a body 107 of a patient 109. The treatment site 106 can include one or more vascular lesions 106A, such as calcified vascular lesions, for example. Additionally, or in the alternative, the treatment site 106 can include vascular lesions 106A, such as fibrous vascular lesions. Still alternatively, in some implementations, the catheter 102 can be used at a treatment site 106 within or adjacent to a heart valve within the body 107 of the patient 109.

The catheter 102 can include an inflatable balloon 104 (sometimes referred to herein simply as a "balloon"), a catheter shaft 110, and a guidewire 112. The balloon 104 can be coupled to the catheter shaft 110. The balloon 104 can include a balloon proximal end 104P and a balloon distal end 104D. The catheter shaft 110 can extend from a proximal portion 114 of the catheter system 100 to a distal portion 116 of the catheter system 100. The catheter shaft 110 can include a longitudinal axis 144. The catheter 102 and/or the catheter shaft 110 can also include a guidewire lumen 118, which is configured to move over the guidewire 112. As utilized herein, the guidewire lumen 118 defines a conduit through which the guidewire 112 extends. The catheter shaft 110 can further include an inflation lumen (not shown) and/or various other lumens for various other purposes. In some embodiments, the catheter 102 can have a distal end opening 120 and can accommodate and be tracked over the guidewire 112 as the catheter 102 is moved and positioned at or near the treatment site 106. In some embodiments, the balloon proximal end 104P can be coupled to the catheter shaft 110, and the balloon distal end 104D can be coupled to the guidewire lumen 118.

The balloon 104 includes a balloon wall 130 that defines a balloon interior 146. The balloon 104 can be selectively inflated with a catheter fluid 132 to expand from a deflated state suitable for advancing the catheter 102 through a patient's vasculature, to an inflated state (as shown in Figure 1) suitable for anchoring the catheter 102 in position relative to the treatment site 106. Stated in another manner, when the balloon 104 is in the inflated state, the balloon wall 130 of the balloon 104 is configured to be positioned substantially adjacent to the treatment site 106. It is appreciated that although Figure 1 illustrates the balloon wall 130 of the balloon 104 being shown spaced apart from the treatment site 106 of the blood vessel 108 when in the inflated state, this is done for ease of illustration. It is recognized that the balloon wall 130 of the balloon 104 will typically be substantially directly adjacent to and/or abutting the treatment site 106 when the balloon 104 is in the inflated state.

The balloon 104 suitable for use in the catheter system 100 includes those that can be passed through the vasculature of a patient 109 when in the deflated state. In some embodiments, the balloons 104 are made from silicone. In other embodiments, the balloon 104 can be made from materials such as polydimethylsiloxane (PDMS), polyurethane, polymers such as PEBAX^{™} material, nylon, or any other suitable material.

The balloon 104 can have any suitable diameter (in the inflated state). In various embodiments, the balloon 104 can have a diameter (in the inflated state) ranging from less than one millimeter (mm) up to 25 mm. In some embodiments, the balloon 104 can have a diameter (in the inflated state) ranging from at least 1.5 mm up to 14 mm. In some embodiments, the balloon 104 can have a diameter (in the inflated state) ranging from at least two mm up to five mm.

In some embodiments, the balloon 104 can have a length ranging from at least three mm to 300 mm. More particularly, in some embodiments, the balloon 104 can have a length ranging from at least eight mm to 200 mm. It is appreciated that a balloon 104 having a relatively longer length can be positioned adjacent to larger treatment sites 106, and, thus, may be usable for imparting pressure waves onto and inducing fractures in larger vascular lesions 106A or multiple vascular lesions 106A at precise locations within the treatment site 106. It is further appreciated that a longer balloon 104 can also be positioned adjacent to multiple treatment sites 106 at any one given time.

The balloon 104 can be inflated to inflation pressures of between approximately one atmosphere (atm) and 70 atm. In some embodiments, the balloon 104 can be inflated to inflation pressures of from at least 20 atm to 60 atm. In other embodiments, the balloon 104 can be inflated to inflation pressures of from at least six atm to 20 atm. In still other embodiments, the balloon 104 can be inflated to inflation pressures of from at least three atm to 20 atm. In yet other embodiments, the balloon 104 can be inflated to inflation pressures from at least two atm to ten atm.

The balloon 104 can have various shapes, including, but not to be limited to, a conical shape, a square shape, a rectangular shape, a spherical shape, a conical/square shape, a conical/spherical shape, an extended spherical shape, an oval shape, a tapered shape, a bone shape, a stepped diameter shape, an offset shape, or a conical offset shape. In some embodiments, the balloon 104 can include a drug-eluting coating or a drug-eluting stent structure. The drug-eluting coating or drug-eluting stent can include one or more therapeutic agents including anti-inflammatory agents, anti-neoplastic agents, anti-angiogenic agents, and the like.

The catheter fluid 132 can be a liquid or a gas. Some examples of the catheter fluid 132 suitable for use can include, but are not limited to one or more of water, saline, contrast medium, fluorocarbons, perfluorocarbons, gases, such as carbon dioxide, or any other suitable catheter fluid 132. In some embodiments, the catheter fluid 132 can be used as a base inflation fluid. In some embodiments, the catheter fluid 132 can include a mixture of saline to contrast medium in a volume ratio of approximately 50:50. In other embodiments, the catheter fluid 132 can include a mixture of saline to contrast medium in a volume ratio of approximately 25:75. In still other embodiments, the catheter fluid 132 can include a mixture of saline to contrast medium in a volume ratio of approximately 75:25. However, it is understood that any suitable ratio of saline to contrast medium can be used. The catheter fluid 132 can be tailored on the basis of composition, viscosity, and the like so that the rate of travel of the pressure waves is appropriately manipulated. In certain embodiments, the catheter fluids 132 suitable for use are biocompatible. A volume of catheter fluid 132 can be tailored by the chosen energy source 124 and the type of catheter fluid 132 used.

In some embodiments, the contrast agents used in the contrast media can include, but are not to be limited to, iodine-based contrast agents, such as ionic or non-ionic iodine-based contrast agents. Some non-limiting examples of ionic iodine-based contrast agents include diatrizoate, metrizoate, iothalamate, and ioxaglate. Some non-limiting examples of non-ionic iodine-based contrast agents include iopamidol, iohexol, ioxilan, iopromide, iodixanol, and ioversol. In other embodiments, non-iodine-based contrast agents can be used. Suitable non-iodine containing contrast agents can include gadolinium (III)-based contrast agents. Suitable fluorocarbon and perfluorocarbon agents can include, but are not to be limited to, agents such as the perfluorocarbon dodecafluoropentane (DDFP, C5F12).

The catheter fluids 132 can include those that include absorptive agents that can selectively absorb light in the ultraviolet region (e.g., at least ten nanometers (nm) to 400 nm), the visible region (e.g., at least 400 nm to 780 nm), or the near-infrared region (e.g., at least 780 nm to 2.5 µm) of the electromagnetic spectrum. Suitable absorptive agents can include those with absorption maxima along the spectrum from at least ten nm to 2.5 µm. Alternatively, the catheter fluids 132 can include those that include absorptive agents that can selectively absorb light in the mid-infrared region (e.g., at least 2.5 µm to 15 µm), or the far-infrared region (e.g., at least 15 µm to one mm) of the electromagnetic spectrum. In various embodiments, the absorptive agent can be those that have an absorption maximum matched with the emission maximum of the laser used in the catheter system 100. By way of non-limiting examples, various lasers usable in the catheter system 100 can include neodymium: yttrium-aluminum-garnet (Nd:YAG - emission maximum = 1064 nm) lasers, holmium:YAG (Ho:YAG - emission maximum = 2.1 µm) lasers, or erbium:YAG (Er:YAG - emission maximum = 2.94 µm) lasers. In some embodiments, the absorptive agents can be water-soluble. In other embodiments, the absorptive agents are not water-soluble. In some embodiments, the absorptive agents used in the catheter fluids 132 can be tailored to match the peak emission of the energy source 124. Various energy sources 124 having emission wavelengths of at least ten nanometers to one millimeter are discussed elsewhere herein.

The catheter shaft 110 of the catheter 102 can be coupled to the one or more energy guides 122A of the energy guide bundle 122 that are in optical communication with the energy source 124. The energy guide(s) 122A can be disposed along the catheter shaft 110 and within the balloon 104. In some embodiments, each energy guide 122A can be an optical fiber, and the energy source 124 can be a laser. The energy source 124 can be in optical communication with the energy guides 122A at the proximal portion 114 of the catheter system 100.

In some embodiments, the catheter shaft 110 can be coupled to multiple energy guides 122A, such as a first energy guide, a second energy guide, a third energy guide, etc., which can be disposed at any suitable positions about and/or relative to the guidewire lumen 118 and/or the catheter shaft 110. For example, in certain non-exclusive embodiments, two energy guides 122A can be spaced apart by approximately 180 degrees about the circumference of the guidewire lumen 118 and/or the catheter shaft 110; three energy guides 122A can be spaced apart by approximately 120 degrees about the circumference of the guidewire lumen 118 and/or the catheter shaft 110; four energy guides 122A can be spaced apart by approximately 90 degrees about the circumference of the guidewire lumen 118 and/or the catheter shaft 110; six energy guides 122A can be spaced apart by approximately 60 degrees about the circumference of the guidewire lumen 118 and/or the catheter shaft 110; eight energy guides 122A can be spaced apart by approximately 45 degrees about the circumference of the guidewire lumen 118 and/or the catheter shaft 110; or ten energy guides 122A can be spaced apart by approximately 36 degrees about the circumference of the guidewire lumen 118 and/or the catheter shaft 110. Still alternatively, multiple energy guides 122A need not be uniformly spaced apart from one another about the circumference of the guidewire lumen 118 and/or the catheter shaft 110. More particularly, it is further appreciated that the energy guides 122A can be disposed uniformly or non-uniformly about the guidewire lumen 118 and/or the catheter shaft 110 to achieve the desired effect in the desired locations.

The catheter system 100 and/or the energy guide bundle 122 can include any number of energy guides 122A in optical communication with the energy source 124 at the proximal portion 114, and with the catheter fluid 132 within the balloon interior 146 of the balloon 104 at the distal portion 116. For example, in some embodiments, the catheter system 100 and/or the energy guide bundle 122 can include from one energy guide 122A to greater than 30 energy guides 122A. Alternatively, in other embodiments, the catheter system 100 and/or the energy guide bundle 122 can include greater than 30 energy guides 122A.

The energy guides 122A can have any suitable design for purposes of generating plasma and/or pressure waves in the catheter fluid 132 within the balloon interior 146. Thus, the general description of the energy guides 122A as light guides is not intended to be limiting in any manner, except for as set forth in the claims appended hereto. More particularly, although the catheter systems 100 are often described with the energy source 124 as a light source, and the one or more energy guides 122A as light guides, the catheter system 100 can alternatively include any suitable energy source 124 and energy guides 122A for purposes of generating the desired plasma in the catheter fluid 132 within the balloon interior 146. For example, in one non-exclusive alternative embodiment, the energy source 124 can be configured to provide high voltage pulses, and each energy guide 122A can include an electrode pair including spaced-apart electrodes that extend into the balloon interior 146. In such embodiment, each pulse of high voltage is applied to the electrodes and forms an electrical arc across the electrodes, which, in turn, generates the plasma and forms the pressure waves in the catheter fluid 132 that are utilized to provide the fracture force onto the vascular lesions 106A at the treatment site 106. Still alternatively, the energy source 124 and/or the energy guides 122A can have another suitable design and/or configuration.

In certain embodiments, the energy guides 122A can include an optical fiber or flexible light pipe. The energy guides 122A can be thin and flexible and can allow light signals to be sent with very little loss of strength. The energy guides 122A can include a core surrounded by a cladding about its circumference. In some embodiments, the core can be a cylindrical core or a partially cylindrical core. The core and cladding of the energy guides 122A can be formed from one or more materials, including but not limited to one or more types of glass, silica, or one or more polymers. The energy guides 122A may also include a protective coating, such as a polymer. It is appreciated that the index of refraction of the core will be greater than the index of refraction of the cladding.

Each energy guide 122A can guide energy along its length from a guide proximal end 122P to the guide distal end 122D having at least one optical window (not shown) that is positioned within the balloon interior 146.

The energy guides 122A can assume many configurations about and/or relative to the catheter shaft 110 of the catheter 102. In some embodiments, the energy guides 122A can run parallel to the longitudinal axis 144 of the catheter shaft 110. In some embodiments, the energy guides 122A can be physically coupled to the catheter shaft 110. In other embodiments, the energy guides 122A can be disposed along a length of an outer diameter of the catheter shaft 110. In yet other embodiments, the energy guides 122A can be disposed within one or more energy guide lumens within the catheter shaft 110.

The energy guides 122A can also be disposed at any suitable positions about the circumference of the guidewire lumen 118 and/or the catheter shaft 110, and the guide distal end 122D of each of the energy guides 122A can be disposed at any suitable longitudinal position relative to the length of the balloon 104 and/or relative to the length of the guidewire lumen 118 to more effectively and precisely impart pressure waves for purposes of disrupting the vascular lesions 106A at the treatment site 106.

In certain embodiments, the energy guides 122A can include one or more photoacoustic transducers 154, where each photoacoustic transducer 154 can be in optical communication with the energy guide 122A within which it is disposed. In some embodiments, the photoacoustic transducers 154 can be in optical communication with the guide distal end 122D of the energy guide 122A. In such embodiments, the photoacoustic transducers 154 can have a shape that corresponds with and/or conforms to the guide distal end 122D of the energy guide 122A.

The photoacoustic transducer 154 is configured to convert light energy into an acoustic wave at or near the guide distal end 122D of the energy guide 122A. The direction of the acoustic wave can be tailored by changing an angle of the guide distal end 122D of the energy guide 122A.

In certain embodiments, the photoacoustic transducers 154 disposed at the guide distal end 122D of the energy guide 122A can assume the same shape as the guide distal end 122D of the energy guide 122A. For example, in certain non-exclusive embodiments, the photoacoustic transducer 154 and/or the guide distal end 122D can have a conical shape, a convex shape, a concave shape, a bulbous shape, a square shape, a stepped shape, a half-circle shape, an ovoid shape, and the like. The energy guide 122A can further include additional photoacoustic transducers 154 disposed along one or more side surfaces of the length of the energy guide 122A.

In some embodiments, the energy guides 122A can further include one or more diverting features or "diverters" (not shown in Figure 1), such as within the energy guide 122A and/or near the guide distal end 122D of the energy guide 122A, that are configured to direct energy from the energy guide 122A toward a side surface which can be located at or near the guide distal end 122D of the energy guide 122A, before the energy is directed toward the balloon wall 130. A diverting feature can include any feature of the system that diverts energy from the energy guide 122A away from its axial path toward a side surface of the energy guide 122A. The energy guides 122A can each include one or more optical windows disposed along the longitudinal or circumferential surfaces of each energy guide 122A and in optical communication with a diverting feature. Stated in another manner, the diverting features can be configured to direct energy in the energy guide 122A toward a side surface that is at or near the guide distal end 122D, where the side surface is in optical communication with an optical window. The optical windows can include a portion of the energy guide 122A that allows energy to exit the energy guide 122A from within the energy guide 122A, such as a portion of the energy guide 122A lacking a cladding material on or about the energy guide 122A.

Examples of the diverting features suitable for use include a reflecting element, a refracting element, and a fiber diffuser. The diverting features suitable for focusing energy away from the tip of the energy guides 122A can include, but are not to be limited to, those having a convex surface, a gradient-index (GRIN) lens, and a mirror focus lens. Upon contact with the diverting feature, the energy is diverted within the energy guide 122A to one or more of a plasma generator 133 and the photoacoustic transducer 154 that is in optical communication with a side surface of the energy guide 122A. When utilized, the photoacoustic transducer 154 then converts light energy into an acoustic wave that extends away from the side surface of the energy guide 122A.

As noted above, in the embodiment illustrated in Figure 1, the system console 123 includes one or more of the energy source 124, the power source 125, the system controller 126, and the GUI 127. Alternatively, the system console 123 can include more components or fewer components than those specifically illustrated in Figure 1. For example, in certain non-exclusive alternative embodiments, the system console 123 can be designed without the GUI 127. Still alternatively, one or more of the energy source 124, the power source 125, the system controller 126, and the GUI 127 can be provided at any suitable location within the catheter system 100 without the specific need for the system console 123.

As shown, the system console 123, and the components included therewith, is operatively coupled to the catheter 102, the energy guide bundle 122, and the remainder of the catheter system 100. For example, in some embodiments, as illustrated in Figure 1, the system console 123 can include a console connection aperture 148 (also sometimes referred to generally as a "socket") by which the energy guide bundle 122 is mechanically coupled to the system console 123. In such embodiments, the energy guide bundle 122 can include a guide coupling housing 150 (also sometimes referred to generally as a "ferrule") that houses a portion, such as the guide proximal end 122P, of each of the energy guides 122A. The guide coupling housing 150 is configured to fit and be selectively retained within the console connection aperture 148 to provide the mechanical coupling between the energy guide bundle 122 and the system console 123.

The energy guide bundle 122 can also include a connector assembly 152 (or "shell") that connects each of the individual energy guides 122A with the system console 123.

The energy source 124 can be selectively and/or alternatively coupled in optical communication with each of the energy guides 122A, such as to the guide proximal end 122P of each of the energy guides 122A, in the energy guide bundle 122. In particular, the energy source 124 is configured to generate energy in the form of a source beam 124A, such as a pulsed source beam, that can be selectively and/or alternatively directed to and received by each of the energy guides 122A in the energy guide bundle 122 as an individual guide beam 124B. Alternatively, the catheter system 100 can include more than one energy source 124. For example, in one non-exclusive alternative embodiment, the catheter system 100 can include a separate energy source 124 for each of the energy guides 122A in the energy guide bundle 122.

The energy source 124 can have any suitable design. In certain embodiments, the energy source 124 can be configured to provide sub-millisecond pulses of energy from the energy source 124 that are focused onto a small spot in order to couple it into the guide proximal end 122P of the energy guide 122A. Such pulses of energy are then directed and/or guided along the energy guides 122A to a location within the balloon interior 146 of the balloon 104, thereby inducing plasma formation in the catheter fluid 132 within the balloon interior 146 of the balloon 104, such as via the plasma generator 133 that can be located at or near the guide distal end 122D of the energy guide 122A. In particular, in such embodiments, the energy emitted at the guide distal end 122D of the energy guide 122A is directed toward and energizes the plasma generator 133 to form the plasma in the catheter fluid 132 within the balloon interior 146. The plasma formation causes rapid bubble formation, and imparts pressure waves upon the treatment site 106. An exemplary plasma-induced bubble 134 is illustrated in Figure 1.

In various non-exclusive alternative embodiments, the sub-millisecond pulses of energy from the energy source 124 can be delivered to the treatment site 106 at a frequency of between approximately one hertz (Hz) and 5000 Hz, between approximately 30 Hz and 1000 Hz, between approximately ten Hz and 100 Hz, or between approximately one Hz and 30 Hz. Alternatively, the sub-millisecond pulses of energy can be delivered to the treatment site 106 at a frequency that can be greater than 5000 Hz or less than one Hz, or any other suitable range of frequencies.

It is appreciated that although the energy source 124 is typically utilized to provide pulses of energy, the energy source 124 can still be described as providing a single source beam 124A, i.e., a single pulsed source beam.

The energy sources 124 suitable for use can include various types of light sources, including lasers and lamps. Alternatively, the energy sources 124 can include any suitable type of energy source.

Suitable lasers can include short pulse lasers on the sub-millisecond timescale. In some embodiments, the energy source 124 can include lasers on the nanosecond (ns) timescale. The lasers can also include short pulse lasers on the picosecond (ps), femtosecond (fs), and microsecond (us) timescales. It is appreciated that there are many combinations of laser wavelengths, pulse widths, and energy levels that can be employed to achieve plasma in the catheter fluid 132 of the catheter 102. In various non-exclusive alternative embodiments, the pulse widths can include those falling within a range including from at least ten ns to 3000 ns, at least 20 ns to 100 ns, or at least one ns to 500 ns. Alternatively, any other suitable pulse width range can be used.

Exemplary nanosecond lasers can include those within the UV to IR spectrum, spanning wavelengths of about ten nanometers (nm) to one millimeter (mm). In some embodiments, the energy sources 124 suitable for use in the catheter systems 100 can include those capable of producing light at wavelengths of from at least 750 nm to 2000 nm. In other embodiments, the energy sources 124 can include those capable of producing light at wavelengths of from at least 700 nm to 3000 nm. In still other embodiments, the energy sources 124 can include those capable of producing light at wavelengths of from at least 100 nm to ten micrometers (µm). Nanosecond lasers can include those having repetition rates of up to 200 kHz.

In some embodiments, the laser can include a Q-switched thulium:yttrium-aluminum-garnet (Tm:YAG) laser. In other embodiments, the laser can include a neodymium:yttrium-aluminum-garnet (Nd:YAG) laser, holmium:yttrium-aluminum-garnet (Ho:YAG) laser, erbium:yttrium-aluminum-garnet (Er:YAG) laser, excimer laser, helium-neon laser, carbon dioxide laser, as well as doped, pulsed, fiber lasers.

In still other embodiments, the energy source 124 can include a plurality of lasers that are grouped together in series. In yet other embodiments, the energy source 124 can include one or more low energy lasers that are fed into a high energy amplifier, such as a master oscillator power amplifier (MOPA). In still yet other embodiments, the energy source 124 can include a plurality of lasers that can be combined in parallel or in series to provide the energy needed to create the plasma bubble 134 in the catheter fluid 132.

The catheter system 100 can generate pressure waves having maximum pressures in the range of at least one megapascal (MPa) to 100 MPa. The maximum pressure generated by a particular catheter system 100 will depend on the energy source 124, the absorbing material, the bubble expansion, the propagation medium, the balloon material, and other factors. In various non-exclusive alternative embodiments, the catheter systems 100 can generate pressure waves having maximum pressures in the range of at least approximately two MPa to 50 MPa, at least approximately two MPa to 30 MPa, or approximately at least 15 MPa to 25 MPa.

The pressure waves can be imparted upon the treatment site 106 from a distance within a range from at least approximately 0.1 millimeters (mm) to greater than approximately 25 mm, extending radially from the energy guides 122A when the catheter 102 is placed at the treatment site 106. In various non-exclusive alternative embodiments, the pressure waves can be imparted upon the treatment site 106 from a distance within a range from at least approximately ten mm to 20 mm, at least approximately one mm to ten mm, at least approximately 1.5 mm to four mm, or at least approximately 0.1 mm to ten mm extending radially from the energy guides 122A when the catheter 102 is placed at the treatment site 106. In other embodiments, the pressure waves can be imparted upon the treatment site 106 from another suitable distance that is different than the foregoing ranges. In some embodiments, the pressure waves can be imparted upon the treatment site 106 within a range of at least approximately two MPa to 30 MPa at a distance from at least approximately 0.1 mm to ten mm. In some embodiments, the pressure waves can be imparted upon the treatment site 106 from a range of at least approximately two MPa to 25 MPa at a distance from at least approximately 0.1 mm to ten mm. Still alternatively, other suitable pressure ranges and distances can be used.

The power source 125 is electrically coupled to and is configured to provide the necessary power to each of the energy source 124, the system controller 126, the GUI 127, and the handle assembly 128. The power source 125 can have any suitable design for such purposes.

The system controller 126 is electrically coupled to and receives power from the power source 125. The system controller 126 is coupled to and is configured to control operation of each of the energy source 124 and the GUI 127. The system controller 126 can include one or more processors or circuits for purposes of controlling the operation of at least the energy source 124 and the GUI 127. For example, the system controller 126 can control the energy source 124 for generating pulses of energy as desired and/or at any desired firing rate.

The system controller 126 can also be configured to control the operation of other components of the catheter system 100, such as the positioning of the catheter 102 adjacent to the treatment site 106, the inflation of the balloon 104 with the catheter fluid 132, etc. Further, or in the alternative, the catheter system 100 can include one or more additional controllers that can be positioned in any suitable manner for purposes of controlling the various operations of the catheter system 100. For example, in certain embodiments, an additional controller and/or a portion of the system controller 126 can be positioned and/or incorporated within the handle assembly 128.

The GUI 127 is accessible by the user or operator of the catheter system 100. The GUI 127 is electrically connected to the system controller 126. With such a design, the GUI 127 can be used by the user or operator to ensure that the catheter system 100 is effectively utilized to impart pressure onto and induce fractures into the vascular lesions 106A at the treatment site 106. The GUI 127 can provide the user or operator with information that can be used before, during, and after using the catheter system 100. In one embodiment, the GUI 127 can provide static visual data and/or information to the user or operator. In addition, or in the alternative, the GUI 127 can provide dynamic visual data and/or information to the user or operator, such as video data or any other data that changes over time during the use of the catheter system 100. In various embodiments, the GUI 127 can include one or more colors, different sizes, varying brightness, etc., that may act as alerts to the user or operator. Additionally, or in the alternative, the GUI 127 can provide audio data or information to the user or operator. The specifics of the GUI 127 can vary depending upon the design requirements of the catheter system 100, or the specific needs, specifications, and/or desires of the user or operator.

As shown in Figure 1, the handle assembly 128 can be positioned at or near the proximal portion 114 of the catheter system 100. In this embodiment, the handle assembly 128 is coupled to the balloon 104 and is positioned spaced apart from the balloon 104. Alternatively, the handle assembly 128 can be positioned at another suitable location.

The handle assembly 128 is attached to the catheter shaft 110 and is handled and used by the user or operator to operate, position, and control the catheter 102. The design and specific features of the handle assembly 128 can vary to suit the design requirements of the catheter system 100. In the embodiment illustrated in Figure 1, the handle assembly 128 is separate from, but in electrical and/or fluid communication with one or more of the system controller 126, the energy source 124, the fluid pump 138, and the GUI 127.

In some embodiments, the handle assembly 128 can integrate and/or include at least a portion of the system controller 126 within an interior of the handle assembly 128. For example, as shown, in certain such embodiments, the handle assembly 128 can include circuitry 156, which is electrically coupled between catheter electronics and the system console 123, and which can form at least a portion of the system controller 126. In one embodiment, the circuitry 156 can include a printed circuit board having one or more integrated circuits, or any other suitable circuitry. In an alternative embodiment, the circuitry 156 can be omitted, or can be included within the system controller 126, which in various embodiments can be positioned outside of the handle assembly 128, such as within the system console 123. It is understood that the handle assembly 128 can include fewer or additional components than those specifically illustrated and described herein.

Further included with the handle assembly 128 is an energy activation member 157 (also sometimes referred to herein simply as an "energy activator"), such as an energy activation button, that can be coupled to the circuitry 156 within the handle assembly 128 which forms a part of the system controller 126. The energy activator 157 is configured to enable the user or operator to selectively activate the catheter system 100 as desired.

In various embodiments, as noted above, the source manifold 136 can be integrated and/or incorporated within the handle assembly 128, and can be positioned at or near the proximal portion 114 of the catheter system 100. As shown, the source manifold 136 can include one or more openings that can receive an inflation conduit 140 that is coupled in fluid communication with the fluid pump 138, the guidewire 112 and/or the guidewire lumen 118, one or more energy guides 122A of the energy guide bundle 122, and/or the catheter shaft 110. More particularly, the source manifold 136 can include one or more of a media inflation port 158, a guidewire lumen port 160, an energy guide port 162, and a catheter shaft port 164.

The catheter system 100 can also include the fluid pump 138 that is configured to inflate the balloon 104 with the catheter fluid 132 as needed.

Various embodiments of the source manifold 136, and the specific components included therewith, are illustrated and described in detail herein below within subsequent Figures.

As with all embodiments illustrated and described herein, various structures may be omitted from the figures for clarity and ease of understanding. Further, the figures may include certain structures that can be omitted without deviating from the intent and scope of the invention.

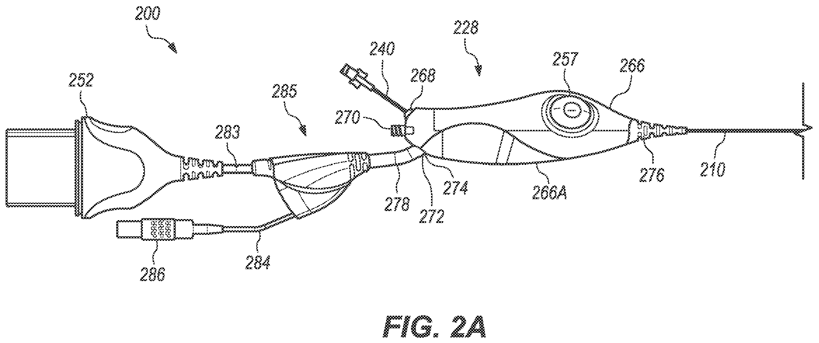

Figure 2A is a simplified schematic view of a portion of an embodiment of the catheter system 200 including a handle assembly 228, a connector assembly 252, and an adapter assembly 285. In all embodiments, each of the handle assembly 228, the connector assembly 252, and the adapter assembly 285 are separated and/or excluded from one another. In other words, in all embodiments, elements of the handle assembly 228, the connector assembly 252, and/or the adapter assembly 285 are mutually exclusive from one another.

The design of the handle assembly 228 and the various components retained therein can be varied to suit the requirements of the catheter system 200. As illustrated in some embodiments, the handle assembly 228 can include the energy activator 257, an assembly housing 266, including and/or defining one or more of an inflation conduit inlet 268, a guidewire inlet 270, and a handle distal outlet 276. Alternatively, the handle assembly 228 can include more components or fewer components than those specifically illustrated and described herein, with the exception of the components of the connector assembly 252, the routing assembly 280, and/or the adapter assembly 285.

In some embodiments, the assembly housing 266 can be formed from two housing members 266A (only one of which is shown in Figure 2A), formed as a first housing side and a second housing side that are selectively coupled together to form the complete assembly housing 266.

The inflation conduit inlet 268 is configured to couple the inflation conduit 240 into the assembly housing 266.

The guidewire inlet 270 is configured to couple the guidewire 212 into the assembly housing 266.

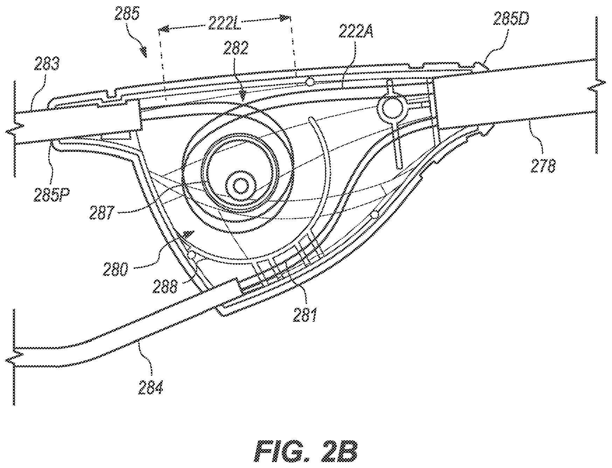

The energy guide inlet 272 is configured to couple the energy guide bundle 122, including the one or more energy guides 222A (illustrated in Figure 2B), into the assembly housing 266.

The electrical inlet 274 is configured to couple an electrical cable 280 (as illustrated in Figure 2B) into the assembly housing 266.

It is appreciated that in certain embodiments, the energy guide inlet 272 and the electrical inlet 274 can be formed together into a single inlet. For example, in one embodiment, the energy guides 222A and an electrical cable 281 (illustrated in Figure 2B) can be shrouded within an optical/electrical cable 278 as the energy guides 222A and the electrical cable 281 enter into the assembly housing 266 through the energy guide inlet 272 and the electrical inlet 274, respectively. Alternatively, the energy guide inlet 272 and the electrical inlet 274 can be formed independently of one another.

The handle distal outlet 276 provides an outlet from the assembly housing 266 for each of the inflation conduit 240, the catheter shaft 210, the guidewire 112 (illustrated in Figure 1), the guidewire lumen 118 (illustrated in Figure), the energy guides 122A (Illustrated in Figure 1), as such components extend toward the balloon 104 (illustrated in Figure 1).

An optical guide 283 can guide the energy guides 222A from the connector assembly 252 to the routing assembly 280. The connector assembly 252 is spaced apart, positioned away, and/or entirely separate from the handle assembly 228 and the adapter assembly 285.

An electrical guide 284 can guide the electrical cable 281 from the electrical connector 286 to the routing assembly 280.

In some embodiments, the adapter assembly 285 can house the components of the routing assembly 280. The adapter assembly 285 can interconnect the optical/electrical cable 278, the optical guide 283, and/or the electrical guide 284. The adapter assembly 285 can include a housing distal end 285D (illustrated in Figure 2B) and a housing proximal end 285P (illustrated in Figure 2B). In some embodiments, the adapter assembly 285 can include a y-adapter. The adapter assembly 285 is spaced apart, positioned away, and/or entirely separate from the handle assembly 228 and the connector assembly 252.

The electrical connector 286 can connect the electrical cable 281 and/or the electrical guide 284 to the handle assembly 228 and/or the adapter assembly 285. The electrical connector 286 can also connect to an external power source (such as the energy source 124, illustrated in Figure 1).

Figure 2B is a simplified schematic cross-sectional view of the embodiment of the adapter assembly 285 in Figure 2A, including a routing assembly 280. As shown in Figure 2B, the adapter assembly 285 can include the optical/electrical cable 278, the electrical cable 281, the routing assembly 280, including an inner routing guide 287, the optical guide 283, and/or the electrical guide 284. The routing assembly 280, the energy guides 222A, the optical/electrical cable 278, the optical guide 283, the electrical guide 284, and/or the adapter assembly 285 shown in Figure 2B can be somewhat similar to the embodiments shown and described herein.

The routing assembly 280 can be configured to reduce the mechanical strain on the energy guides 222A and/or the electrical cable 281. The routing assembly 280 can route the energy guides 222A and/or the electrical cable 281 in any suitable direction throughout the catheter system 200. The specifics of the routing assembly 280 can vary depending upon the design requirements of the catheter system 200, or the specific needs, specifications, and/or desires of the user or operator. It is appreciated that the routing assembly 280 can include additional or fewer elements than illustrated in Figure 2B. The routing assembly 280 can be positioned within any suitable location in the catheter system 100.

In all embodiments, the routing assembly 280 is spaced apart from and/or outside of the handle assembly 228. The optical/electrical cable 278 can be coupled between the routing assembly 280 and the handle assembly 228. The guide distal end 122D (illustrated in Figure 1) and/or the guide proximal end 122P (illustrated in Figure 1) can be fixed relative to the routing assembly 280. In some embodiments, the guide distal end 122D is movable relative to the routing assembly 280.

The electrical cable 281 guides power from the electrical connector 286 (illustrated in Figure 2A) through the optical/electrical cable 278, the electrical guide 284, the adapter assembly 285 to the handle assembly 228 (illustrated in Figure 2A). The electrical cable 281 can connect the handle assembly 228 to any suitable source of power (such as the energy source 124, illustrated in Figure 1).

The routing assembly 280 can include a routing space 282. The routing space 282 defines a space for the routing, positioning, and/or movement of the energy guides 222A inside of the routing assembly 280. Movement of the energy guides 222A throughout the catheter system 100 and/or the routing space 282 can result in alternating periods of slack and tension on the energy guides 222A, which can cause axial and longitudinal movement of the guides and cables. Such movement may result in the undesired cramping and/or twisting of the energy guides 222A of the catheter system 200 and may cause the degradation of the energy guides 222A.

The routing space 282 retains a routing length 222L of each of the energy guides 222A. The routing length 222L includes the length of the energy guides 222A that is positioned within the routing space 282 during operation and use of the catheter system 200. The routing length 222L is a portion of the total length of the energy guides 222A.. In the various embodiments illustrated by Figure 2B, the routing length 222L is shown with dashed lines to illustrate the routing length 222L that is inside of the routing assembly 280. The routing space 282 can be configured so that the routing length 222L of at least one of the energy guides 222A is adjustable.

The inner routing guide 287 can be positioned in and/or within the routing space 282. The routing length 222L of each of the energy guides can be positioned about, around, and/or near the inner routing guide 287.

In certain embodiments, the energy guides 222A can loop greater than approximately 5 degrees around the inner routing guide 287 and less than approximately 1080 degrees around the inner routing guide 287. The energy guides 222A can loop approximately 5 degrees, 10 degrees, 15 degrees, 20 degrees, 25 degrees, 30 degrees, 35 degrees, 40 degrees, 45 degrees, 50 degrees, 55 degrees, 60 degrees, 65 degrees, 70 degrees, 75 degrees, 80 degrees, 85 degrees, 90 degrees, 95 degrees, 100 degrees, 105 degrees, 110 degrees, 115 degrees, 120 degrees, 125 degrees, 130 degrees, 135 degrees, 140 degrees, 145 degrees, 150 degrees, 155 degrees, 160 degrees, 165 degrees, 170 degrees, 175 degrees, 180 degrees, 185 degrees, 190 degrees, 195 degrees, 200 degrees, 205 degrees, 210 degrees, 215 degrees, 220 degrees, 225 degrees, 230 degrees, 235 degrees, 240 degrees, 245 degrees, 250 degrees, 255 degrees, 260 degrees, 265 degrees, 270 degrees, 275 degrees, 280 degrees, 285 degrees, 290 degrees, 295 degrees, 300 degrees, 305 degrees, 310 degrees, 315 degrees, 320 degrees, 325 degrees, 330 degrees, 335 degrees, 340 degrees, 345 degrees, 350 degrees, 355 degrees, 360 degrees, 365 degrees, 370 degrees, 375 degrees, 380 degrees, 385 degrees, 390 degrees, 395 degrees, 400 degrees, 405 degrees, 410 degrees, 415 degrees, 420 degrees, 425 degrees, 430 degrees, 435 degrees, 440 degrees, 445 degrees, 450 degrees, 455 degrees, 460 degrees, 465 degrees, 470 degrees, 475 degrees, 480 degrees, 485 degrees, 490 degrees, 495 degrees, 500 degrees, 505 degrees, 510 degrees, 515 degrees, 520 degrees, 525 degrees, 530 degrees, 535 degrees, 540 degrees, 545 degrees, 550 degrees, 555 degrees, 560 degrees, 565 degrees, 570 degrees, 575 degrees, 580 degrees, 585 degrees, 590 degrees, 595 degrees, 600 degrees, 605 degrees, 610 degrees, 615 degrees, 620 degrees, 625 degrees, 630 degrees, 635 degrees, 640 degrees, 645 degrees, 650 degrees, 655 degrees, 660 degrees, 665 degrees, 670 degrees, 675 degrees, 680 degrees, 685 degrees, 690 degrees, 695 degrees, 700 degrees, 705 degrees, 710 degrees, 715 degrees, 720 degrees, 725 degrees, 730 degrees, 735 degrees, 740 degrees, 745 degrees, 750 degrees, 755 degrees, 760 degrees, 765 degrees, 770 degrees, 775 degrees, 780 degrees, 785 degrees, 790 degrees, 795 degrees, 800 degrees, 805 degrees, 810 degrees, 815 degrees, 820 degrees, 825 degrees, 830 degrees, 835 degrees, 840 degrees, 845 degrees, 850 degrees, 855 degrees, 860 degrees, 865 degrees, 870 degrees, 875 degrees, 880 degrees, 885 degrees, 890 degrees, 895 degrees, 900 degrees, 905 degrees, 910 degrees, 915 degrees, 920 degrees, 925 degrees, 930 degrees, 935 degrees, 940 degrees, 945 degrees, 950 degrees, 955 degrees, 960 degrees, 965 degrees, 970 degrees, 975 degrees, 980 degrees, 985 degrees, 990 degrees, 995 degrees, 1000 degrees, 1005 degrees, 1010 degrees, 1015 degrees, 1020 degrees, 1025 degrees, 1030 degrees, 1035 degrees, 1040 degrees, 1045 degrees, 1050 degrees, 1055 degrees, 1060 degrees, 1065 degrees, 1070 degrees, 1075 degrees, or 1080 degrees about the inner routing guide. In various embodiments, the energy guides 222A can loop less than approximately 5 degrees around the inner routing guide 287 and greater than approximately 1080 degrees about the inner routing guide 287.

The inner routing guide 282 can guide at least one of the movement and positioning of the energy guides 222A within the routing assembly 280. The inner routing guide 287 can reduce the mechanical strain on the energy guides 222A, various cables, guides, and/or other components within the routing assembly 280. As shown in Figure 2B, the routing space 282 includes the routing length 222L of the energy guides 222A, in order to reduce the mechanical strain and/or mechanical forces that may impact the routing length 222L of the energy guides 222A.

The adapter assembly 285 can cooperate with the routing space 282 so that a user can service the energy guides 222A. For example, the adapter assembly 285 can be at least partially removable to expose the energy guides 222A and/or the routing space 282 so that an operator can service, repair, and/or remove the energy guides 222A (or any other component of the routing assembly 280).

The inner routing guide 287 and/or the routing space 282 can (i) provide a storage area for the energy guides 222A and/or (ii) maintain the organization of the energy guides 222A. The specifics of the inner routing guide 287 can vary depending upon the design requirements of the catheter system 100, the handle assembly 228, the routing assembly 280, and/or the specific needs, specifications, and/or desires of the user or operator.

The inner routing guide 287 can position components such as the energy guides 222A. For example, as shown in Figure 2B, the inner routing guide 287 can position the energy guides 222A in a somewhat circular pattern. It is appreciated that the inner routing guide 287 can take the form of any suitable number of geometric structures at any suitable sizes. The inner routing guide 287 is shown as a circle in Figure 2B merely for ease of understanding. The inner routing guide 287 can cooperate with an outer routing guide 288 to define a path that is able to receive a portion of the energy guides 222A so that the portion can be positioned around, about, and/or near the inner routing guide 287.

The outer routing guide 288 can be substantially similar to the inner routing guide 287 in form and/or function. While the outer routing guide 288 is illustrated in Figure 2B as an arc, it is appreciated that the outer routing guide 288 can take the form of any suitable number of geometric structures at any suitable sizes.

The inner routing guide 287 and/or the outer routing guide 288 can be configured to enable movement of the guide distal end 122D relative to the routing assembly 280 due to changes in the routing length 222L stored in the routing space 282.

In certain embodiments, the inner routing guide 287 and/or the outer routing guide 288 increases the likelihood that the routing length 222L remains in a looped orientation such that tension or axial movement experienced by the energy guides 222A does not cause the energy guides 222A to kink or otherwise bend at an undesirable angle. This reduces the possibility of damage to the energy guides 222A.

In various embodiments, the inner routing guide 287 and/or the outer routing guide 288 can include a post or other structure having a width, thereby maintaining a spacing or diameter of the routing length 222L positioned within the routing space 282. The width of the inner routing guide 287 and/or the outer routing guide 288 may be larger than a bend or kink radius of the energy guides 222A, which may depend on the particular material from which the energy guides 222A are constructed.

During use of the catheter system 100 of the present technology during a medical procedure, the energy guides 222A, such as optical fibers, are positioned into the adapter assembly 285 through the optical guide 283 into the routing space 282 around, about, and/or near the inner routing guide 287. Subsequently, the energy guides 222A are directed out of the adapter assembly 285 toward the optical/electrical cable 278 in combination with the electrical cable 281 at the housing distal end 285D. In addition, the electrical cable 281, can be (i) directed through the optical/electrical cable 278 via the adapter assembly 285, (ii) directed through the routing assembly 280, and/or (iii) directed out of the adapter assembly 285 and towards the housing distal end 285D through the optical/electrical cable 278.

During the medical procedure, should the energy guides 222A experience an axial movement and/or a change in tension, for example, due to inflation of the balloon 104 (illustrated in Figure 1) or similar structure, the routing length 222L of the energy guides 222A can be positioned in a loop within the routing space 282, i.e., an increase in the diameter of the looped portion will occur as shown in FIG. 2B, thereby reducing the likelihood of kinking, as well as reducing any excess force on the energy guides 222A that may cause a shift in positioning. Alternatively, should the inner routing guide 287 be rotatably coupled to the housing, the routing length 222L of the energy guides 222A may be taken up by rotating the inner routing guide 287 either automatically or manually. Should the adapter assembly 285 include a substantially rounded shape, the diameter of the expanded loop-portion of the energy guides 222A may expand to a width of the housing without contacting an angled surface which could cause kinking or bending of the energy guides 222A. In addition, the separate inlet portions of the optical guide 283 and the electrical guide 284 allow for separate and independent routing of the energy guides 222A and the electrical cable 281, allowing for ease of connectivity and manipulation of the energy guides 222A and/or the electrical cable 281 and their connection points.

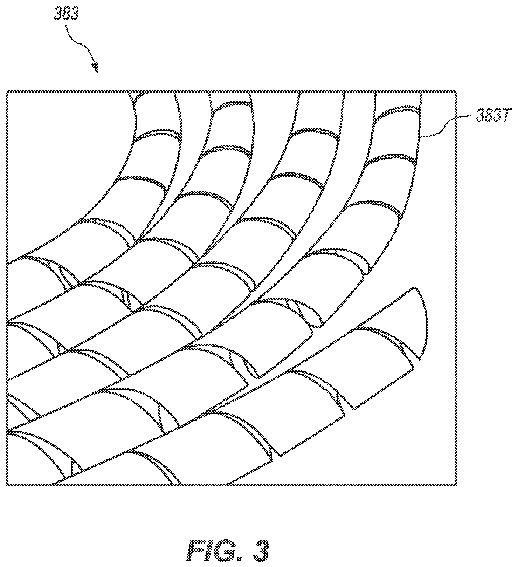

Figure 3 is a perspective view of an embodiment of an optical guide 383 utilized by various embodiments of the energy guides 222A (illustrated in Figure 2B), the routing assembly 280 (illustrated in Figure 2B), and the adapter assembly 285 (for example, as illustrated in Figure 2B).

The optical guide 383 can guide and/or protect the energy guides 222A (illustrated in Figure 2B) from damage. The optical guide 383 can include a flexible cable that reduces spring energy build-up when the guides and/or cables are coiled or bent. The specifics of the optical guide 383 can vary depending upon the design requirements of the catheter system 200, the handle assembly 228, the routing assembly 280, the adapter assembly 285, and/or the specific needs, specifications and/or desires of the user or operator.

The optical guide 383 can include a protector tubing 383T. The protector tubing 383T can be configured to allow the free movement of the guides and/or cables within the optical guide 383. In some embodiments, the protector tubing 383T can be spiral-cut (such as shown in the various embodiments illustrated by Figure 3). In various embodiments, the protector tubing 383T can be at least partially formed from Polytetrafluoroethylene and/or any suitable polymer. The optical guide 383 and/or the protector tubing 383T can be at least partially formed from light-absorbing materials (with or without fillers) that at least partially inhibit and/or absorb unwanted light and/or energy from escaping from the optical guide 383 and/or the protector tubing 383T in the event of damage to the energy guides 222A and/or electrical cables 281.

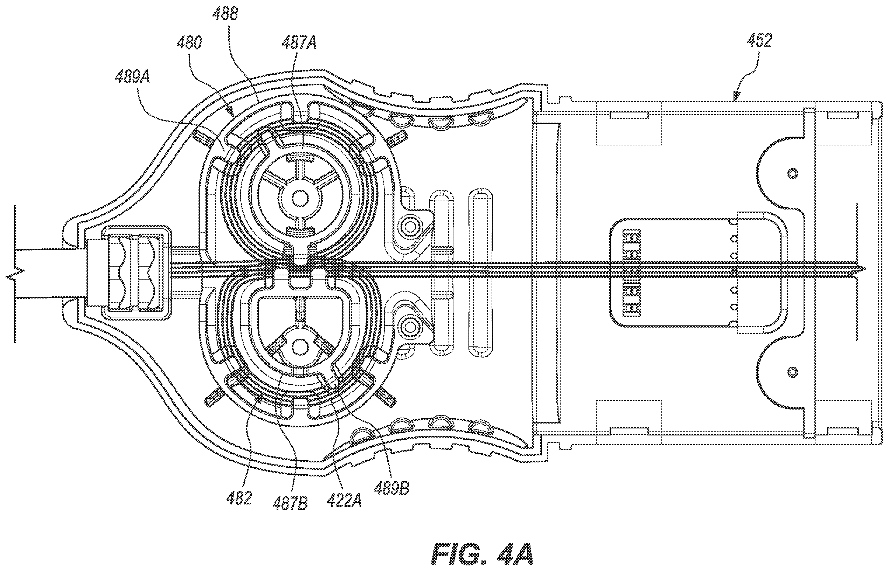

Figure 4A is a simplified schematic cross-sectional view of an embodiment of the connector assembly 452, including the routing assembly 480.

In some embodiments, the routing assembly 480 may include an outer routing guide 488 for routing the routing length 222L (for example, as illustrated in Figure 2B), thereby positioning the energy guides 422A between the inner routing guide 487 and the outer routing guide 488 in the routing space 482. The outer routing guide 488 can be displaced from the inner routing guide 487, about which the energy guides 422A are positioned around, about, and/or near. The inner routing guide 487 and/or the outer routing guide 488 cooperate to define the positioning and/or the routing of the individual energy guides 422A through the routing assembly 480.

The specifics of outer routing guide 488 can vary depending upon the design requirements of the catheter system 100 (illustrated in Figure 1), the connector assembly 452, the routing assembly 480, and/or the specific needs, specifications and/or desires of the user or operator.

Between the inner routing guide 487 and the outer routing guide 488 the routing assembly 480, for example, may include a channel, groove, depression, aperture, or similar passage in the body of routing assembly 480, between the inner routing guide 487 and the outer routing guide 488. As shown in the various embodiments illustrated by Figure 4A, this space between the inner routing guide 487 and the outer routing guide 488 may define multiple paths for receiving the routing length 222L of the energy guides 422A to aid in managing and positioning the energy guides 422A through the length of the catheter system 100 to reduce the likelihood of tangling, kinking, or the like to the energy guides 422A.

The inner routing guide 487 and/or the outer routing guide 488 can also include a guide retainer 489A. The guide retainer 489A can retain the energy guides 422A between the inner routing guide 487 and the outer routing guide 488 and reduces the likelihood that the energy guides 422A exit the routing space 482 during the operation of the catheter system 100, including the movement of the energy guides 422A. The design of the guide retainer 489A can vary depending on the design requirements of the routing assembly 480, the inner routing guide 487, and/or the outer routing guide 488. While the guide retainers 489A, 489B are shown in Figure 4A as specific rectangular shapes, it is appreciated that any suitable geometric structure of any suitable size that is capable of retaining the individual energy guides 422A within the routing space 482 may be used.

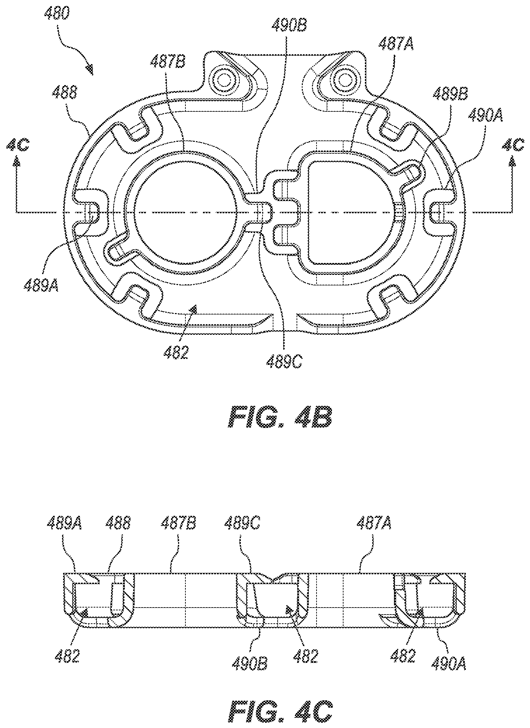

Figure 4B is a simplified schematic cross-sectional view of the routing assembly 480 in Figure 4A. In some embodiments, such as illustrated in Figures 4A-4B, a plurality of guide retainers 489A, 489B are spaced apart from one another along the path of the energy guides 422A within the routing space 482 (illustrated Figure 4A). For example, as illustrated in the embodiments illustrated by Figure 4A, the energy guides 422A can be retained by the guide retainers of 489A, 489B in a somewhat infinity loop and/or figure eight orientation in the routing space 482 about two inner routing guides 487A, 487B. This orientation limits the amount of mechanical strain placed on the energy guides 422A as they move within the routing space 482. It is appreciated that the inner routing guides 487A, 487B and/or the outer routing guide 488 can include any suitable number, size, structure, and/or distribution of the guide retainers 489A, 489B depending on the design requirements of the routing assembly 480.

In the embodiment illustrated by Figures 4A-4B, the inner routing guides 487A, 487B have varying geometric shapes and sizes to accommodate the infinity loop and/or figure eight orientation of the routing length 222L (illustrated in Figure 2B) of the energy guides 222A in the routing space 482. For example, in the various embodiments illustrated by Figures 4A-4B, one inner routing guide 487 is somewhat O-shaped, and the other inner routing guide 487 is somewhat D-shaped.

The routing assembly 480 can also include an assembly aperture 490A. In certain embodiments, the assembly aperture 490A can be formed in various locations of the routing assembly 480. For example, in the embodiments illustrated in Figure 4B, a plurality of assembly apertures 480A, 490B, are positioned directly below corresponding guide retainers 489A.

The positioning of the assembly aperture 490A allows for the accommodation of additional routing length 222L of energy guides 422A and/or reduced stress/tension applied to the guide retainers 489A, 489B when the energy guides 422A are positioned therebetween. In other words, when the energy guides 422A are received by the outer routing guide 488 and retained by the guide retainers 489A, 489B, the assembly apertures 490A, 490B provide space for the movement of the energy guides 422A whilst retaining the position of the energy guides 422A in the routing space 482.

For example, as shown in Figures 4A and 4B, the portion of the routing length 222L positioned toward the center of the routing assembly 480 can become crowded due to a crossing and/or looping of the energy guides 422A. The assembly aperture 490A positioned in the center area of the routing assembly 480 can have an increased area to accommodate this crowding. In addition, the inner routing guides 487A, 487B can include multiple guide retainers 489C that are directly positioned over the central assembly aperture 490B.

Figure 4C is a cross-sectional view of the routing assembly 480 taken on lines 4C-4C in Figure 4B. As shown in Figure 4C, the routing assembly 480 can include cross-sectional spaces defined between the inner routing guides 487A, 487B, the inner routing guide 487A and the outer routing guide 488, and/or the inner routing guide 487B and the outer routing guide 488. These cross-sectional spaces are illustrated as substantially u-shaped in Figure 4C, but it is understood that any suitable number of cross-sectional spaces, shapes, and/or sizes can be used to define the routing space 482 illustrated in the various embodiments illustrated by Figures 4A-4C.

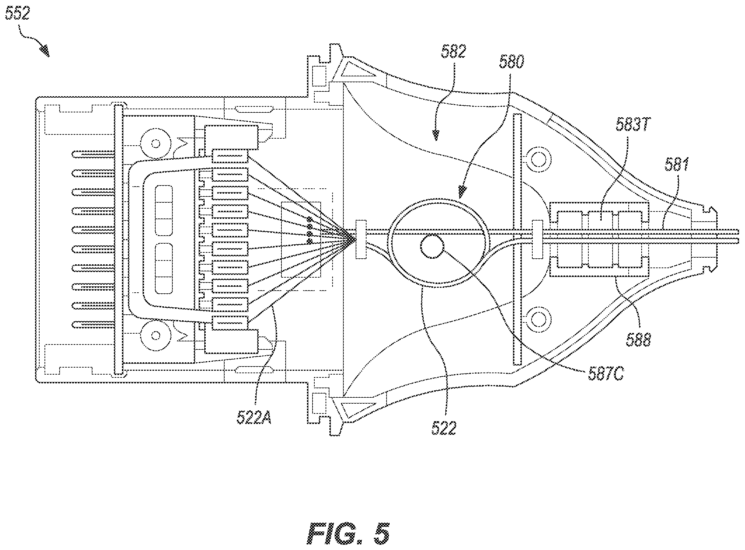

Figure 5 is a simplified top view illustration of an embodiment of the connector assembly 552. More specifically, Figure 5 illustrates various internal components and features that can be included in various embodiments of the connector assembly 552. As shown in Figure 5, in certain embodiments, the connector assembly 552 can internally include a portion of the one or more energy guides 522A (e.g., the routing length 222L illustrated in Figure 2B) and a routing assembly 580.

In various embodiments, the connector assembly 552 can be configured to provide strain relief as it brings each of the individual energy guides 522A closer together so that the energy guides 522A and/or the energy guide bundle 522 can be in a more compact form as it extends with the catheter 102 (illustrated in Figure 1) into the blood vessel 108 (illustrated in Figure 1) during use of the catheter system 100 (illustrated in Figure 1). For ease of understanding, in some embodiments, such as shown in Figure 5, the plurality of individual energy guides 522A is shown as one energy guide bundle 522 for ease of understanding. It is appreciated that the individual energy guides 522A do not necessarily require bundling, such as shown in various embodiments illustrated by Figure 4A.

The design of the individual energy guides 522A can be varied. For example, as shown in Figure 5, in certain embodiments, each of the individual energy guides 522A and/or energy guide bundle 522 can include a protector tube 583 (also illustrated in Figure 3) within which all of the energy guides 522A are maintained as the energy guides 522A extend with the catheter 102 toward the balloon 104 (illustrated in Figure 1). The connector assembly 552 can also include a locking crimp 588 that is configured to tightly bunch the energy guides 522A together in a controlled manner to form the energy guide bundle 522.

In some embodiments, the connector assembly 552 can be at least partially removable to expose the energy guides 522A and/or the routing space 582 so that an operator can service, repair, and/or remove the energy guides 522A (or any other component of the connector assembly 552).

The inner routing guide 587C can position components such as the routing length 222L (illustrated in Figure 2B) of the energy guides 522A in the routing space 582. For example, as shown in Figure 5, the inner routing guide 587C can position the routing length 222L of the energy guides 522A in a somewhat circular pattern. It is appreciated that the inner routing guide 587C can take the form of any suitable geometric structure and that the inner routing guide 587C is shown as a circle in Figure 5 merely for ease of understanding. The inner routing guide 587C can define a path that is able to receive the routing length 222L of the energy guides 522A so that the routing length 222L can form a loop around the inner routing guide 587C. The inner routing guide 587C can be configured to enable movement of the guide distal end 122D (illustrated in Figure 1) relative to the connector assembly 552.

In certain embodiments, the inner routing guide 587C increases the likelihood that a portion of the energy guides 522A (e.g., the routing length 222L) remains in a looped orientation such that tension or axial movement experienced by the energy guides 522A does not cause the energy guides 522A to kink or otherwise bend at an undesirable angle. This reduces the possibility of damage to the energy guides 522A.

The inner routing guide 587C can include a post or other structure having a width, thereby maintaining a spacing or diameter of the portion of the energy guides 522A located within the routing space 582. The width of the inner routing guide 587C may be larger than a bend or kink radius of the energy guides 522A, which may depend on the particular material from which the energy guides 522A are constructed.

In some embodiments, the routing assembly 580 may also include an outer routing guide 488 (for example, as shown in Figure 4 with respect to the routing assembly 480) for receiving a portion of the energy guides 522A, thereby positioning the energy guides 522A within the routing space 582. The outer routing guide 488 can be displaced from a portion of the inner routing guide 587C, about which the energy guides 522A have looped around, thereby providing a spacing between the energy guides 522A within the connector assembly 552. The outside housing of the connector assembly 552 can also function as the outer routing guide 488, depending on the design requirements of the routing assembly 580.

As provided herein, in various embodiments, the catheter systems and related methods disclosed herein can include a catheter configured to advance to a vascular lesion, such as a calcified vascular lesion or a fibrous vascular lesion, at a treatment site located within or adjacent to a blood vessel within a body of a patient. The catheter includes a catheter shaft, and an inflatable balloon that is coupled and/or secured to the catheter shaft. The balloon can include a balloon wall that defines a balloon interior. The balloon can be configured to receive a catheter fluid within the balloon interior to expand from a deflated state suitable for advancing the catheter through a patient's vasculature, to an inflated state suitable for anchoring the catheter in position relative to the treatment site.

In certain embodiments, the catheter systems and related methods utilize an energy source, e.g., a light source such as a laser or another suitable energy source, which provides energy that is guided by one or more energy guides, e.g., light guides such as optical fibers, which are disposed along the catheter shaft and within the balloon interior of the balloon to create a localized plasma in the catheter fluid that is retained within the balloon interior of the balloon. The energy guide can be used in conjunction with a plasma generator that is positioned at or near a guide distal end of the energy guide within the balloon interior of the balloon located at the treatment site. The creation of the localized plasma can initiate a pressure wave and can initiate the rapid formation of one or more bubbles that can rapidly expand to a maximum size and then dissipate through a cavitation event that can launch a pressure wave upon collapse. The rapid expansion of the plasma-induced bubbles (also sometimes referred to simply as "plasma bubbles") can generate one or more pressure waves in the catheter fluid retained within the balloon interior of the balloon and thereby impart pressure waves onto and induce fractures in the vascular lesions at the treatment site within or adjacent to the blood vessel wall within the body of the patient. In some embodiments, the energy source can be configured to provide sub-millisecond pulses of energy, e.g., light energy, to initiate the plasma formation in the catheter fluid within the balloon to cause the rapid bubble formation and to impart the pressure waves upon the balloon wall at the treatment site. Thus, the pressure waves can transfer mechanical energy through an incompressible catheter fluid to the treatment site to impart a fracture force on the intravascular lesion. Without wishing to be bound by any particular theory, it is believed that the rapid change in catheter fluid momentum upon the balloon wall that is in contact with the intravascular lesion is transferred to the intravascular lesion to induce fractures to the lesion.

The catheter systems and related methods disclosed herein can further include a handle assembly that is attached to the catheter shaft, and that is handled and used by the user or operator to operate, position, and control the catheter. In various embodiments, the handle assembly has a source manifold incorporated and/or integrated therein. In such embodiments, the source manifold can include one or more of a manifold housing, a pressure sensor that is coupled to and/or integrated into the manifold housing, and a media inflation port, a guidewire lumen port, an energy guide port, and a catheter shaft port that is formed into and/or coupled to the manifold housing. The pressure sensor is configured to sense a fluid pressure of the catheter fluid within the catheter system. For example, in certain embodiments, the pressure sensor can be configured to sense a fluid pressure within the balloon interior or at any desired location along an inflation conduit. The media inflation port is configured to couple the inflation conduit into and/or through the manifold housing so that the catheter fluid can be directed as desired through the handle assembly and into the balloon interior. The guidewire lumen port is configured to couple a guidewire lumen, which defines a conduit through which a guidewire extends, into, from, and/or through the manifold housing, so that the guidewire lumen can thus extend from the handle assembly into and/or through the balloon interior. The energy guide port is configured to couple the one or more energy guides into and/or through the manifold housing, so that the energy guides can guide energy from the energy source, through the handle assembly, and into the balloon interior. The catheter shaft port is configured to couple the catheter shaft to the manifold housing so that the user can effectively control the positioning of the catheter shaft, with the balloon attached thereto, substantially adjacent to the vascular lesion(s) at the treatment site via manipulation of the handle assembly.

It should be noted that, as used in this specification and the appended claims, the singular forms "a," "an," and "the" include plural referents unless the content and/or context clearly dictates otherwise. It should also be noted that the term "or" is generally employed in its sense, including "and/or" unless the content or context clearly dictates otherwise.

It should also be noted that, as used in this specification and the appended claims, the phrase "configured" describes a system, apparatus, or other structure that is constructed or configured to perform a particular task or adopt a particular configuration. The phrase "configured" can be used interchangeably with other similar phrases such as arranged and configured, constructed and arranged, constructed, manufactured and arranged, and the like.

The headings used herein are provided for consistency with suggestions under 37 CFR 1.77 or otherwise to provide organizational cues. These headings shall not be viewed to limit or characterize the invention(s) set out in any claims that may issue from this disclosure. As an example, a description of a technology in the "Background" is not an admission that technology is prior art to any invention(s) in this disclosure. Neither is the "Summary" or "Abstract" to be considered as a characterization of the invention(s) set forth in issued claims.

The embodiments described herein are not intended to be exhaustive or to limit the invention to the precise forms disclosed in the following detailed description. Rather, the embodiments are chosen and described so that others skilled in the art can appreciate and understand the principles and practices. As such, aspects have been described with reference to various specific and preferred embodiments and techniques. However, it should be understood that many variations and modifications may be made while remaining within the spirit and scope herein.

It is understood that although a number of different embodiments of the catheter systems have been illustrated and described herein, one or more features of any one embodiment can be combined with one or more features of one or more of the other embodiments, provided that such combination satisfies the intent of the present invention.

While a number of exemplary aspects and embodiments of the catheter systems have been discussed above, those of skill in the art will recognize certain modifications, permutations, additions, and sub-combinations thereof. It is therefore intended that the following appended claims and claims hereafter introduced are interpreted to include all such modifications, permutations, additions, and sub-combinations as are within their scope, and no limitations are intended to the details of construction or design herein shown.

## Claims

1. A catheter system (100) for treating a treatment site (106) within or adjacent to a vessel wall (108A) or a heart valve within a body of a patient, the catheter system (100) including a first energy guide and a second energy guide, each of the energy guides (122A) including a guide distal end (122D) and a guide proximal end (122P), the catheter system (100) comprising:
a routing assembly (280) that defines a routing space (282) that retains a routing length (222L) of each of the energy guides (222A), the routing assembly (280) including an inner routing guide (287) that is positioned within the routing space (282), the routing length (222L) of each of the energy guides (122A) being positioned at least partially about the inner routing guide (287), the routing space (282) being configured so that the routing length (222L) of at least one of the energy guides (222A) is adjustable, the guide distal end (122D) being movable relative to the routing assembly (280).

2. The catheter system (100) of claim 1 wherein each of the energy guides (122A) includes an optical fiber.

3. The catheter system (100) of any of claims 1-2 wherein the guide proximal end (122P) is fixed relative to the routing assembly (280).

4. The catheter system (100) of any of claims 1-3 wherein each energy guide (122A) is positioned at least approximately 90 degrees about the inner routing guide (287).

5. The catheter system (100) of any of claims 1-4 further comprising an adapter assembly (285) that houses the inner routing guide (287).

6. The catheter system (100) of any of claims 1-5 further comprising an electrical cable (281) that is positioned about the inner routing guide (287).

7. The catheter system (100) of any of claims 1-6 wherein each energy guide (122A) includes an optical guide (283) that is configured to protect each energy guide (122A).

8. The catheter system (100) of any of claims 1-7 further comprising a handle assembly (228) that is usable by a user to selectively position the energy guides (122A) near the treatment site (106), the routing assembly (280) being positioned remotely from the handle assembly (228).

9. The catheter system (100) of any of claims 1-8 further comprising a connector assembly (252) that is configured to bring the energy guides (122A) closer together, the routing assembly (280) being positioned within the connector assembly (252).

10. The catheter system (100) of any of claims 1-9 wherein the inner routing guide (287) cooperates with an outer routing guide (288) to define a path that is able to receive a portion of the energy guides (122A).

11. The catheter system (100) of claim 10 wherein the inner routing guide (287) and the outer routing guide (288) maintain one of a space and a diameter of the routing length (222L) positioned within the routing space (282).

12. The catheter system (100) of any of claims 10-11 wherein the inner routing guide (287) and the outer routing guide (288) increase a likelihood that the routing length (222L) remains in a looped orientation.

13. The catheter system (100) of any of claims 10-12 wherein one of the inner routing guide (287) and the outer routing guide (288) includes a guide retainer (489A) that retains the energy guides (122A) between the inner routing guide (287) and the outer routing guide (288).

14. The catheter system (100) of claim 13 wherein the guide retainer (489A) is configured to reduce a likelihood that the energy guides (122A) exit the routing space (282) during operation of the catheter system (100).

15. The catheter system (100) of any of claims 10-14 wherein the inner routing guide (287) and the outer routing guide (288) define multiple paths for receiving the routing length (222L).

## Patentansprüche

1. Kathetersystem (100) zur Behandlung einer Behandlungsstelle (106) innerhalb oder benachbart zu einer Gefäßwand (108A) oder einer Herzklappe im Körper eines Patienten, wobei das Kathetersystem (100) eine erste Energieführung und eine zweite Energieführung aufweist, wobei jede der Energieführungen (122A) ein distales Führungsende (122D) und ein proximales Führungsende (122P) aufweist, wobei das Kathetersystem (100) aufweist:
eine Führungsanordnung (280), die einen Führungsraum (282) definiert, der eine Führungslänge (222L) von jeder der Energieführungen (222A) aufnimmt, wobei die Führungsanordnung (280) eine innere Führung (287) aufweist, die innerhalb des Führungsraums (282) positioniert ist, wobei die Führungslänge (222L) von jeder der Energieführungen (122A) zumindest teilweise um die innere Führung (287) angeordnet ist, wobei der Führungsraum (282) so konfiguriert ist, dass die Führungslänge (222L) von mindestens einer der Energieführungen (222A) einstellbar ist, und wobei das distale Führungsende (122D) relativ zur Führungsanordnung (280) beweglich ist.

2. Kathetersystem (100) nach Anspruch 1, wobei jede der Energieführungen (122A) eine optische Faser aufweist.

3. Kathetersystem (100) nach einem der Ansprüche 1 bis 2, wobei das proximale Führungsende (122P) relativ zur Führungsanordnung (280) fixiert ist.

4. Kathetersystem (100) nach einem der Ansprüche 1 bis 3, wobei jede Energieführung (122A) in einem Winkel von mindestens annähernd 90 Grad um die inneren Führung (287) angeordnet ist.

5. Kathetersystem (100) nach einem der Ansprüche 1 bis 4, das ferner eine Adapteranordnung (285) aufweist, die die innere Führung (287) aufnimmt.

6. Kathetersystem (100) nach einem der Ansprüche 1 bis 5, das ferner ein elektrisches Kabel (281) aufweist, das um die innere Führung (287) angeordnet ist.

7. Kathetersystem (100) nach einem der Ansprüche 1 bis 6, wobei jede Energieführung (122A) eine optische Führung (283) aufweist, die konfiguriert ist, eine jeweilige Energieführung (122A) zu schützen.

8. Kathetersystem (100) nach einem der Ansprüche 1 bis 7, das ferner eine Griffanordnung (228) aufweist, die von einem Anwender nutzbar ist, um die Energieführungen (122A) selektiv in der Nähe der Behandlungsstelle (106) zu positionieren, wobei die Führungsanordnung (280) entfernt von der Griffanordnung (228) angeordnet ist.

9. Kathetersystem (100) nach einem der Ansprüche 1 bis 8, das ferner eine Verbinderanordnung (252) aufweist, die konfiguriert ist, die Energieführungen (122A) näher zusammenzubringen, wobei die Führungsanordnung (280) innerhalb der Verbinderanordnung (252) angeordnet ist.

10. Kathetersystem (100) nach einem der Ansprüche 1 bis 9, wobei die innere Führung (287) mit einer äußeren Führung (288) zusammenwirkt, um einen Weg zu definieren, der geeignet ist, einen Abschnitt der Energieführungen (122A) aufzunehmen.

11. Kathetersystem (100) nach Anspruch 10, wobei die innere Führung (287) und die äußere Führung (288) entweder einen Raum oder einen Durchmesser der innerhalb des Führungsraums (282) angeordneten Führungslänge (222L) aufrechterhalten.

12. Kathetersystem (100) nach einem der Ansprüche 10 bis 11, wobei die innere Führung (287) und die äußere Führung (288) die Wahrscheinlichkeit erhöhen, dass die Führungslänge (222L) in einer geschlungenen Ausrichtung verbleibt.

13. Kathetersystem (100) nach einem der Ansprüche 10 bis 12, wobei die innere Führung (287) oder die äußere Führung (288) eine Führungshalterung (489A) aufweist, die die Energieführungen (122A) zwischen der inneren Führung (287) und der äußeren Führung (288) hält.

14. Kathetersystem (100) nach Anspruch 13, wobei die Führungshalterung (489A) so konfiguriert ist, dass sie die Wahrscheinlichkeit verringert, dass die Energieführungen (122A) während des Betriebs des Kathetersystems (100) aus dem Führungsraum (282) austreten.

15. Kathetersystem (100) nach einem der Ansprüche 10 bis 14, wobei die innere Führung (287) und die äußere Führung (288) mehrere Wege zur Aufnahme der Führungslänge (222L) definieren.

## Revendications

1. Système de cathéter (100) pour traiter un site de traitement (106) dans ou à proximité d'une paroi de vaisseau (108A) ou d'une valve cardiaque dans un corps d'un patient, le système de cathéter (100) incluant un premier guide d'énergie et un second guide d'énergie, chacun des guides d'énergie (122A) incluant une extrémité distale de guide (122D) et une extrémité proximale de guide (122P), le système de cathéter comprenant:
un ensemble de routage (280) qui définit un espace de routage (282) qui retient une longueur de routage (222L) de chacun des guides d'énergie (222A), l'ensemble de routage (280) incluant un guide de routage interne (287) qui est positionné dans l'espace de routage (282), la longueur de routage (222L) de chacun des guides d'énergie (122A) étant positionnée au moins partiellement autour du guide de routage interne (287), l'espace de routage (282) étant configuré de sorte que la longueur de routage (222L) d'au moins l'un des guides d'énergie (222A) est ajustable, l'extrémité distale de guide (122D) étant mobile par rapport à l'ensemble de routage (280).

2. Système de cathéter (100) selon la revendication 1, dans lequel chacun des guides d'énergie (122A) inclut une fibre optique.

3. Système de cathéter (100) selon l'une quelconque des revendications 1 à 2, dans lequel l'extrémité proximale de guide (122P) est fixe par rapport à l'ensemble de routage (280).

4. Système de cathéter (100) selon l'une quelconque des revendications 1 à 3, dans lequel chaque guide d'énergie (122A) est positionné à au moins approximativement 90 degrés autour du guide de routage interne (287).

5. Système de cathéter (100) selon l'une quelconque des revendications 1 à 4, comprenant en outre un ensemble adaptateur (285) qui loge le guide de routage interne (287).

6. Système de cathéter (100) selon l'une quelconque des revendications 1 à 5 comprenant en outre un câble électrique (281) qui est positionné autour du guide de routage interne (287).

7. Système de cathéter (100) selon l'une quelconque des revendications 1 à 6, dans lequel chaque guide d'énergie (122A) inclut un guide optique (283) qui est configuré pour protéger chaque guide d'énergie (122A).

8. Système de cathéter (100) selon l'une quelconque des revendications 1 à 7 comprenant en outre un ensemble de poignée (228) qui est utilisable par un utilisateur pour positionner sélectivement les guides d'énergie (122A) à proximité du site de traitement (106), l'ensemble de routage (280) étant positionné à distance de l'ensemble de poignée (228).

9. Système de cathéter (100) selon l'une quelconque des revendications 1 à 8 comprenant en outre un ensemble de connecteur (252) qui est configuré pour rapprocher les guides d'énergie (122A), l'ensemble de routage (280) étant positionné à l'intérieur de l'ensemble de connecteur (252).

10. Système de cathéter (100) selon l'une quelconque des revendications 1 à 9, dans lequel le guide de routage interne (287) coopère avec un guide de routage externe (288) pour définir une voie qui est capable de recevoir une partie des guides d'énergie (122A).

11. Système de cathéter (100) selon la revendication 10, dans lequel le guide de routage interne (287) et le guide de routage externe (288) maintiennent l'un d'un espace et d'un diamètre de la longueur de routage (222L) positionnée à l'intérieur de l'espace de routage (282).

12. Système de cathéter (100) selon l'une quelconque des revendications 10 à 11 dans lequel le guide de routage interne (287) et le guide de routage externe (288) augmentent une probabilité que la longueur de routage (222L) reste dans une orientation en boucle.

13. Système de cathéter (100) selon l'une quelconque des revendications 10 à 12, dans lequel l'un du guide de routage interne (287) et du guide de routage externe (288) inclut un dispositif de retenue de guide (489A) qui retient les guides d'énergie (122A) entre le guide de routage interne (287) et le guide de routage externe (288).

14. Système de cathéter (100) selon la revendication 13, dans lequel le dispositif de retenue de guide (489A) est configuré pour réduire une probabilité que les guides d'énergie (122A) sortent de l'espace de routage (282) pendant le fonctionnement du système de cathéter (100).

15. Système de cathéter (100) selon l'une quelconque des revendications 10 à 14, dans lequel le guide de routage interne (287) et le guide de routage externe (288) définissent de multiples voies pour recevoir la longueur de routage (222L).
